# EUROPÄISCHE PATENTSCHRIFT

(11) **EP 0 983 988 B1**
(45) Veröffentlichungstag und Bekanntmachung des Hinweises auf die Patenterteilung: **23.01.2002**
(21) Anmeldenummer: 99117101.8
(22) Anmeldetag: 31.08.1999
(51) Int. Cl.: C07C 45/67, C07C 49/203

(54) **Kontinuierliches Verfahren zur Herstellung von ungesättigten Ketonen**
Continuous process for the preparation of unsaturated ketones
Procédé continu pour la préparation de cétones insaturées

(30) Priorität: 07.09.1998 DE 19840747
(43) Veröffentlichungstag der Anmeldung: 08.03.2000
(73) Patentinhaber: BASF AKTIENGESELLSCHAFT, 67056 Ludwigshafen (DE)
(72) Erfinder: Oost, Carsten, Dr., 67098 Bad Dürkheim (DE); Stroezel, Manfred, 68549 Ilvesheim (DE); Etzrodt, Heinz, Dr., 67434 Neustadt (DE); Weller, Dietmar, Dr., 67067 Ludwigshafen (DE); Bockstiegel, Bernhard, Dr., 67354 Römerberg (DE); Reimer, Klaus, 67112 Mutterstadt (DE); Kaibel, Gerd, Dr., 68623 Lampertheim (DE); Jaedicke, Hagen, Dr., 67069 Ludwigshafen (DE)

(56) Entgegenhaltungen:
- EP-A- 0 022 955
- FR-A- 2 371 411

## Beschreibung

Die vorliegende Erfindung betrifft ein verbessertes Verfahren zur kontinuierlichen Herstellung von ungesättigten Ketonen durch Umsetzen von α,β-ungesättigten Alkoholen mit Acetessigsäurealkylestern in einer Carroll-Reaktion.

Die Herstellung von ungesättigten Ketonen durch Umsetzung von α,β-ungesättigten Alkoholen mit Acetessigsäurealkylestern in Gegenwart von organischen Aluminiumverbindungen unter Abspaltung des aus dem Acetessigester stammenden Alkohols ist, sieht man von den erfindungsgemäßen Verbesserungen ab, in ihren wesentlichen Merkmalen bereits bekannt. Die unkatalysierte Umsetzung zwischen einem ungesättigten Alkohol und einem Acetessigsäurealkylester wurde erstmals von M.F. Carroll [J. Chem. Soc. (London) 1940, Seiten 704 bis 706] beschrieben. Über den Anwendungsbereich und den Mechanismus dieser Reaktion wurde ein Jahr später von demselben Autor [J. Chem. Soc. (London) 1941, Seiten 507 bis 511] berichtet.

Eine Verfahrensvorschrift für die Herstellung von 6,10,14-Trimethyl-5-pentadecen-2-on durch Umesterung von Acetessigsäureethylester mit 3,7,11-Trimethyl-1-dodecen-3-ol in Anwesenheit von Aluminiumtrialkoholaten ist der französischen Patentschrift 1 219 166 zu entnehmen. Gemäß diesem Verfahren werden die Reaktanten und der Katalysator gemeinsam in der Reaktionsblase vorgelegt und die Reaktion unter destillativer Abtrennung des freiwerdenden Alkohols diskontinuierlich durchgeführt. Dabei wird das gewünschte Keton in einer Reaktionszeit von etwa 10 Stunden in 77 %iger Ausbeute erhalten.

Für eine technische Synthese unbefriedigend an diesem Verfahren sind sowohl die verhältnismäßig langen Reaktionszeiten als auch die unzureichenden Ausbeuten. Die unzureichenden Ausbeuten sind besonders gravierend bei der Herstellung von höheren Ketonen, d.h. bei Verwendung von höheren Alkoholen der Formel II, da diese mit wachsender Kettenlänge zunehmend teurer in ihrer Herstellung werden. Versucht man die Ausbeuten dadurch zu verbessern, daß man die billigere Komponente, hier den Acetessigsäurealkylester, im Überschuß verwendet, so kommt es leicht zur Bildung von Dehydracetsäure als Nebenprodukt, die einerseits den Katalysator desaktiviert und andererseits aus dem gewünschten Produkt nur schwer wieder abzutrennen ist. Zudem kann die Dehydracetsäure auskristallisieren und dadurch die Ablaufleitungen der verwendeten Kolonnen verstopfen.

Es ist eine Reihe weiterer Patentschriften bekannt, in denen verschiedene Varianten dieser sogenannten Carroll'schen Reaktion beschrieben werden. So heißt es in der US-PS 2 795 617 bzw. DE-AS 1 053 498 bzw. CH-PS 342947, daß "obschon es in der Regel weder notwendig noch erwünscht sei, ein Lösungsmittel verwendet werden kann, um den exothermen Reaktionsverlauf zu mildern". Nach diesen Patentschriften wird das Aluminiumtrialkoholat zu dem Acetoacetat des α,β-ungesättigten Alkohols gegeben und die Mischung bei starkem Rühren unter Rückfluß erhitzt, wobei Ausbeuten bis zu 80 % erreicht werden. Die Herstellung des entsprechenden Acetoacetats muß in einer vorangehenden Stufe erfolgen. In der US-PS 2 839 579 bzw. DE-PS 1078112 wird berichtet, daß die thermische Umlagerung der Acetoacetate in einem Lösungsmittel durchgeführt werden kann. Die Herstellung der entsprechenden Acetoacetate erfolgt durch Kondensation von Diketen mit einem entsprechenden ungesättigten Alkohol in einer separaten Stufe. Auch in der DE-PS 1 068 696 heißt es, daß die Mitverwendung eines Lösungsmittels vorteilhaft sein könnte. In allen Fällen werden hochsiedende Lösungsmittel genannt, deren Siedepunkte weit oberhalb der Reaktionstemperatur liegen.

Die in diesen Patenten angegebenen Ausbeuten sind unbefriedigend für eine technische Anwendung. Auch die Mitverwendung eines hochsiedenden Lösungsmittels bringt im allgemeinen keine nennenswerten Ausbeutesteigerungen mit sich und führt daher zu einer Reduzierung der Raumzeitausbeuten. Von erheblichem Nachteil ist es, daß zur Herstellung der Acetoacetate des α,β-ungesättigten Alkohols eine weitere Verfahrensstufe erforderlich ist, da hiermit weitere Kosten verbunden sind.

Ein Verfahren zur Herstellung von 2-Methyl-2-hepten-6-on wird in der DE-AS 2 652 863 beschrieben. Hierbei werden Acetessigsäurealkylester, 3-Methyl-1-buten-3-ol (Methylbutenol) sowie der Katalysator in einem Reaktionsgefäß mit aufgesetzter Fraktionierkolonne vorgelegt und anschließend ein Gemisch aus Acetessigsäurealkylester und Methylbutenol zudosiert. Während der Reaktion soll der Gehalt an Acetessigsäurealkylester im Reaktionsgemisch nicht mehr als 15 Gew.-% betragen, um Nebenreaktionen zu vermeiden. Ein Nachteil dieses Verfahren ist jedoch, daß ein einfaches Eindosieren von Acetessigsäurealkylester in überschüssiges Methylbutenol nicht möglich ist, da der Siedepunkt von Methylbutenol weit unterhalb der Reaktionstemperatur liegt. Die Verwendung eines hochsiedenden Lösungsmittels erniedrigt hingegen die Raumzeitausbeute. Die Ausbeute von 90 % ist unbefriedigend.

In dem Tschechischen Patent 216 360 wird beschrieben, daß die Carroll-Reaktion in einem Gemisch aus ungesättigtem Keton und Acetessigsäuremethylester bzw. -ethylester unter Zugabe einer gerade zur Reaktion erforderlichen Menge des ungesättigten Alkohols durchgeführt wird. Dabei wird aus dem Reaktionsgemisch das Kohlendioxid und ein Gemisch aus dem nicht umgesetzten ungesättigten Alkohol und Methanol bzw. Ethanol abdestilliert, das kontinuierlich in einer angekoppelten Destillationskolonne fraktioniert wird. Der α,β-ungesättigte Alkohol, dessen Siedepunkt unter 180°C liegen muß, wird anschließend in die Reaktion zurückgeführt. Es werden bei Reaktionszeiten von 8 Stunden Ausbeuten von ca. 80 % erreicht. Diese Vorgehensweise ist nach Ausführungen in diesem Patent von Vorteil, weil ein Mitreißen der beiden tiefersiedenden Komponenten aus dem Reaktionsgemisch durch das entstehende Kohlendioxid nicht vermieden werden kann. Die in diesem Patent beschriebene Ankopplung einer Destillationskolonne an das eigentliche Reaktorsystem ist nicht unbedingt erforderlich, da durch richtige Auslegung des Reaktorsystems ein Mitreißen des α,β-ungesättigten Alkohols durch Kohlendioxid vermieden werden kann. Beispielsweise gelingt es gemäß dem Verfahren von DE 2 928 944, nur Methanol und Kohlendioxid abzutrennen und den α,β-ungesättigten Alkohol in der Reaktionsblase zu halten. Somit resultieren aus der angekoppelten Destillationskolonne in erster Linie zusätzliche Investitions- und Energiekosten. Zudem sind die Ausbeuten und Reaktionszeiten bei diesem Verfahren unbefriedigend. Ein weiterer Nachteil des Verfahrens ist die Beschränkung der Siedetemperatur des α,β-ungesättigten Alkohols auf unter 180°C, weil die meisten für die Vitamin-E-Synthese relevanten Alkohole oberhalb von 200°C sieden.

Im Gegensatz zu den oben genannten Patentschriften wird in der DE 2 928 944 die Verwendung eines Lösungsmittels beschrieben, dessen Siedepunkt zwischen dem eingesetzten Acetessigsäureester und dem des hieraus abzuspaltenden Alkohols liegt. Dieses Lösungsmittel wird als "Zwischensieder" bezeichnet. Als mögliche inerte Zwischensieder werden entsprechend siedende Alkohole, Ester, Ether, halogenierte Kohlenwasserstoffe und aromatische Kohlenwasserstoffe genannt; vorzugsweise jedoch aliphatische Ketone mit 4 bis 7 C-Atomen. Als besonders vorteilhafte Ausführungsform wird die Verwendung von 3-Methyl-1-buten-3-ol als reaktiver Zwischensieder genannt, wobei als zusätzliche erwünschte Nebenreaktion dessen Umsetzung mit dem Acetessigsäurealkylester zu 2-Methyl-2-hepten-6-on als weiterem Wertprodukt stattfindet. Als Vorteile der Verwendung eines solchen Zwischensieders werden erhöhte Produktausbeuten (ca. 95 % bezogen auf den Alkohol und ca. 85 % bezogen auf den Acetessigester) sowie kürzere Reaktionszeiten (ca. 4 bis 5 h) und damit hohe Raumzeitausbeuten genannt. Als Reaktorsystem wird für eine diskontinuierliche Reaktionsführung eine Destillationsblase mit aufgesetzter Fraktionierkolonne und für eine kontinuierliche Reaktionsführung eine beheizte Kesselkaskade vorgeschlagen.

Die Verwendung eines Zwischensieders bringt jedoch nicht nur Vorteile mit sich, sondern auch die folgenden Nachteile. Bei Verwendung eines inerten Zwischensieders verringert sich das für die Edukte zur Verfügung stehende Reaktorvolumen, d.h. die erzielbare Raumzeitausbeute verringert sich zwangsläufig. Die Verwendung des reaktiven Zwischensieders 3-Methyl-1-buten-3-ol führt dagegen zu einer Zwangskoppelproduktion von 2-Methyl-2-hepten-6-on, die unerwünscht sein kann. Darüber hinaus ist das Verfahren auf Systeme beschränkt, bei denen der α,β-ungesättigte Alkohol höher siedet als der verwendete Acetessigsäurealkylester. Das in der Patentschrift vorgeschlagene kontinuierliche Verfahren erfordert eine Reaktorkaskade und damit hohe Investitionskosten. Ein weiterer Nachteil ist die relativ geringe Ausbeute bezogen auf den Acetessigsäurealkylester.

Es war daher die Aufgabe der Erfindung, die Umsetzung von α,β-ungesättigten Alkoholen mit Acetessigsäurealkylestern zu ungesättigten Ketonen unter Vermeidung der Nachteile der Verfahren des Standes der Technik durchzuführen. Das neue Verfahren sollte also kontinuierlich in einem einzigen Reaktor durchführbar sein, wobei höhere Produktausbeuten, bezogen auf den ungesättigten Alkohol, und insbesondere höhere Produktausbeuten, bezogen auf den Acetessigsäurealkylester, bei möglichst kurzen Reaktionszeiten und ohne Verwendung eines Zwischensieders erreicht werden sollten. Eine Beschränkung bezüglich der Siedepunkte der Reaktanten sollte dabei nicht bestehen.

Insbesondere sollten die als Riechstoffe und Zwischenprodukte für das Vitamin-E-Vorprodukt Isophytol begehrten Ketone, wie 6,10-Dimethyl-5,9-undecadien-2-on (Geranylaceton), 6,10,14-Trimethyl-5,9,13-pentadecatrien-2-on (Farnesylaceton), 6,10-Dimethyl-5-undecen-2-on (Dihydrogeranylaceton), 6,10,14-Trimethyl-5,10-pentadecadien-2-on (Dihydrofarnesylaceton) und 2-Methyl-2-hepten-6-on kontinuierlich in einem Reaktor mit höheren Ausbeuten und mit höheren Raumzeitausbeuten hergestellt werden können.

Es wurde nun gefunden, daß sich die ungesättigten Ketonen der allgemeinen Formel I 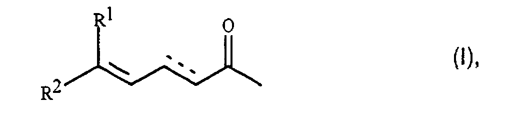 in der die gestrichelte Linie eine zusätzliche C-C-Bindung bedeuten kann, R¹ für eine verzweigte oder unverzweigte Alkylgruppe mit 1 bis 4 C-Atomen steht, und R² für einen gesättigten oder ungesättigten, verzweigten oder unverzweigten, gegebenenfalls durch eine oder mehrere Methoxygruppen substituierten aliphatischen Kohlenwasserstoffrest mit 1 bis 37 C-Atomen, eine Cycloalkylgruppe mit 4 bis 12 C-Atomen oder eine Cycloalkylalkylgruppe mit 5 bis 30 C-Atomen steht, vorzugsweise für eine Gruppe der allgemeinen Formel VI 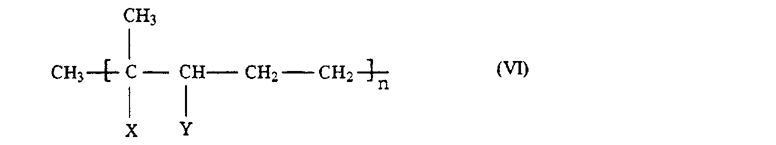 steht, in der n für eine ganze Zahl von 1 bis 6 steht und X und Y entweder beide für H stehen, oder X für Methoxy und Y für H stehen, oder aber X und Y zusammen eine zusätzliche Bindung zwischen den X und Y tragenden C-Atomen bedeuten, durch Umsetzen eines ungesättigten Alkohols der allgemeinen Formel II 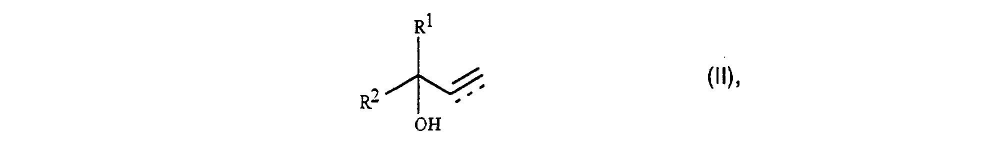 in der R¹ und R² die oben angegebene Bedeutung haben, mit einem Acetessigsäurealkylester der allgemeinen Formel III 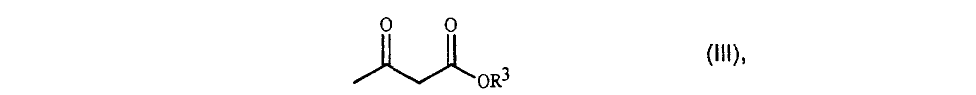 in der R³ für eine verzweigte oder unverzweigte Alkylgruppe mit 1 bis 5 C-Atomen steht, in Gegenwart organischer Aluminiumverbindungen als Katalysator kontinuierlich in einem einzigen Reaktor mit höheren Produktausbeuten und höheren Raumzeitausbeuten herstellen lassen, wenn man
A. den ungesättigten Alkohol der Formel II mit dem Acetessigester der Formel III auf den Einbauten (16) einer Fraktionierkolonne (4) zu dem Acetoacetat des ungesättigten Alkohols der Formel II umsetzt und den dabei entstehenden Alkohol der allgemeinen Formel IV

   R³-OH (IV)

   mit dem Kopfstrom (5) der Kolonne abtrennt und
B. das gebildete Acetoacetat des Alkohols der Formel II im unteren Teil der Fraktionierkolonne und/oder im Sumpf (20) der Fraktionierkolonne in Gegenwart der organischen Aluminiumverbindung in das ungesättigte Keton der Formel I umlagert, wobei das sich hierbei bildende Kohlendioxid über die Fraktionierkolonne (4) in den Kopfstrom (5) gelangt und das gebildete ungesättigte Keton der Formel I über den Sumpf (20) der Kolonne abgetrennt wird.

Mit dem erfindungsgemäßen Verfahren werden Selektivitäten von bis zu 99 % bezogen auf den Alkohol und 97 % bezogen auf den Acetessigsäurealkylester erreicht. Das bedeutet, daß bei einer möglichen Rückführung des nicht umgesetzten Alkohols und Acetessigsäurealkylesters Gesamtausbeuten von nahezu 100 % erzielt werden können. Der Umsatz liegt bei 95 % bezogen auf den Alkohol und bei 98 % bezogen auf den Acetessigsäurealkylester, so daß nur geringe Mengen an Edukt zurückgeführt werden müssen. Die Raumzeitausbeute ist um den Faktor 3 größer als bei den bisher bekannten Verfahren. Zudem läßt sich durch das erfindungsgemäße Verfahren die unerwünschte weitere Umsetzung der ungesättigten Ketone der Formel I, d.h. der Reaktionsprodukte, durch Meerwein-Ponndorf-Verley-Reduktion zu den entsprechenden Alkoholen vermeiden.

Das erfindungsgemäße Verfahren läßt sich prinzipiell auf alle bekannten Varianten der sogenannten Carroll'schen Reaktion anwenden, wobei keine Beschränkung bezüglich der Siedepunkte der Reaktanten besteht. Besondere Bedeutung besitzt das Verfahren jedoch für die Synthese solcher Ketone, die zur Herstellung von Riechstoffen und Isophytol benötigt werden, wie Geranylaceton, Farnesylaceton, Dihydrogeranylaceton, Dihydrofarnesylaceton und 2-Methyl-2-hepten-6-on.

Ein großer Vorteil des erfindungsgemäßen Verfahrens ist, daß die erzielbaren hohen Ausbeuten in einem kontinuierlichen Verfahren in einem Reaktor bei nahezu quantitativem Umsatz bezogen auf alle Einsatzkomponenten erzielt werden, und zwar auch dann, wenn gar kein Überschuß oder ein nur geringer Überschuß einer der Reaktionskomponenten eingesetzt wird. Weiterhin ist es ein großer Vorteil, daß durch das erfindungsgemäße Verfahren die Raumzeitausbeuten der bisher bekannten Verfahren um den Faktor 3 gesteigert werden können.

Von besonderer Bedeutung ist das erfindungsgemäße Verfahren für die Umsetzung von Alkoholen der allgemeinen Formel (IIa) 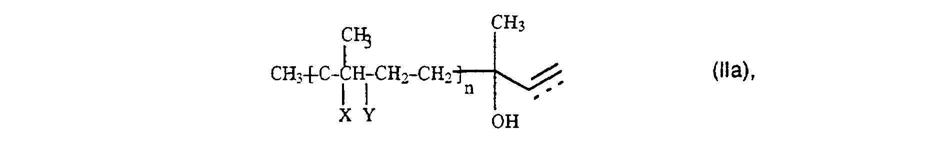 in der x, y und n die oben angegebene Bedeutung haben, wie beispielsweise 3,7-Dimethyl-1,6-octadien-3-ol (Linalool), 3,7-Dimethyl-1-octen-3-ol, 3,7,11-Trimethyl-1,6,10-dodecatrien-3-ol (Nerolidol), 3,7,11-Trimethyl-l-dodecen-3-ol und 3,7,11-Trimethyl-1,6-dodecadien-3-ol (Dihydronerolidol).

Die Reaktion gelingt prinzipiell mit beliebigen Acetessigsäurealkylestern, jedoch werden der Methylester, der Ethylester und der Isopropylester sowohl aus wirtschaftlichen als auch aus verfahrenstechnischen Gründen bevorzugt, da die hieraus abzuspaltenden Alkohole besonders niedrig sieden und so leicht aus dem Reaktionsgemisch entfernt werden können. Die Verwendung von Acetessigsäure-tert.-butylester bringt den Vorteil einer schnelleren Umsetzung und die Vermeidung von Nebenprodukten mit sich. Die Einsatzmengen der Reaktanten wählt man mit Vorteil so, daß sich ein Molverhältnis von Alkohol der Formel II zu Acetessigsäurealkylester der Formel III zwischen 0,8 und 1,2, vorzugsweise von 0,95 bis 1,10 ergibt.

Als organische Aluminiumverbindungen kommen für das erfindungsgemäße Verfahren im wesentlichen Verbindungen der allgemeinen Formel V 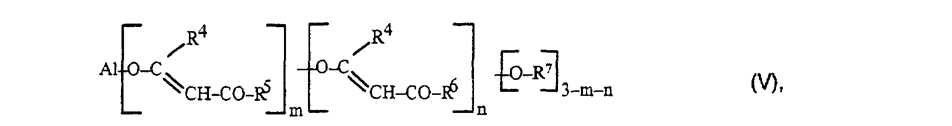 in der R⁴ verzweigte oder unverzweigte Alkyl- oder Alkoxygruppen mit 1 bis 4 C-Atomen, vorzugsweise Methyl- oder Ethylgruppen bedeutet, R⁵ und R⁶ verzweigte oder unverzweigte Alkyl- oder Alkoxygruppen mit 1 bis 5 C-Atomen, vorzugsweise eine Methyl- oder eine 2-Butyl-gruppe bedeuten, R⁷ für eine verzweigte oder unverzweigte Alkylgruppe mit 1 bis 4 C-Atomen steht und m sowie n eine ganze Zahl von 0 bis 3 bedeuten können, wobei n+m ≤ 3 ist, sowie Aluminiumtriaryloxylate in Betracht. Besonders bevorzugt werden flüssige Aluminiumverbindungen, insbesondere Aluminiumverbindungen, bei denen R⁵ ein Methylrest, R⁶ ein Butylrest und die Summe aus n+m = 3 und das Verhältnis n/m > 0,3 ist.

Bei den erstgenannten Katalysatoren handelt es sich also um niedere Aluminiumtrialkoholate wie Aluminiumtrimethylat, -triethylat, -triisopropylat, -tri-sek.-butylat sowie um Verbindungen, die bei der Umsetzung der genannten Aluminiumtrialkoholate mit stöchiometrischen Mengen von Acetylacetonat, Alkylacetoacetat oder Alkylmalonat unter Alkoholabspaltung und Umesterung gebildet werden. Genannt seien beispielsweise Aluminium-triacetoacetat, Aluminium-triacetylacetonat, Aluminiummonoacetoacetat-diethylat, Aluminium-monoacetoacetat-diiso-propylat, Aluminium-diacetoacetat-monoisopropylat.

Bevorzugt verwendet man die Aluminiumtrialkoholate, insbesondere das Aluminiumtriisopropylat sowie das Aluminium-tri-sek.-butylat. Ganz besonders bevorzugt verwendet man gemischte Aluminiumtriacetoacetate, die man durch Umsetzung von Aluminium-sek.-butylat oder Aluminiumtriisopropylat mit Acetessigsäuremethylester unter Abspaltung von 2-Butanol bzw. iso-Propanol und Umesterung der Methoxygruppen mit dem freigesetzten 2-Butanol oder iso-Propanol erhält, wobei der Umesterungsgrad über 30 % betragen soll.

Als Aluminiumtriaryloxylate bezeichnen wir die Aluminiumsalze von aromatischen Hydroxyverbindungen wie Aluminiumtriphenolat, Aluminiumtrikresolate, Aluminiumtrixylenolate, Aluminiumtrinaphtholate, deren Arylreste auch durch niedere Alkyl- oder Alkyloxygruppen, d.h. Alkyl- oder Alkyloxygruppen mit 1 bis 4 C-Atomen, Hydroxygruppen oder Phenyl substituiert sein können. Mit besonderem Vorteil verwendet man hiervon das relativ leicht zugängliche Aluminiumtriphenolat.

Es ist von Vorteil, flüssige Katalysatoren oder Lösungen von festen Katalysatoren zu verwenden und diese in flüssiger Form in die Fraktionierkolonne, bevorzugt in den Sumpf (20) der Kolonne, einzuspeisen. So können zum Beispiel Aluminiumtrialkoholate in Acetessigsäurealkylester oder einer Mischung aus Acetessigsäurealkylester und einem Alkohol der allgemeinen Formel II gelöst verwendet werden.

Die Menge der Aluminiumverbindung wird allgemein so bemessen, daß ihre Konzentration im Reaktionsgemisch 0,05 Gew.-% Al nicht unterschreitet und zu Beginn der Reaktion 6 Gew.-% Al nicht überschreitet. Bezogen auf umzusetzenden Acetessigsäurealkylester werden im allgemeinen 0,5 bis 5 mol-% an der Aluminiumverbindung benötigt. Für das bevorzugt verwendete Aluminiumtriisopropylat und das oben beschriebene gemischte, aus Aluminium-sek.-butylat und Acetessigsäuremethylester hergestellte Aluminiumtriacetoacetat werden z.B. Mengen von etwa 1 bis 3 mol-% bezogen auf den umzusetzenden Acetessigsäurealkylester eingesetzt.

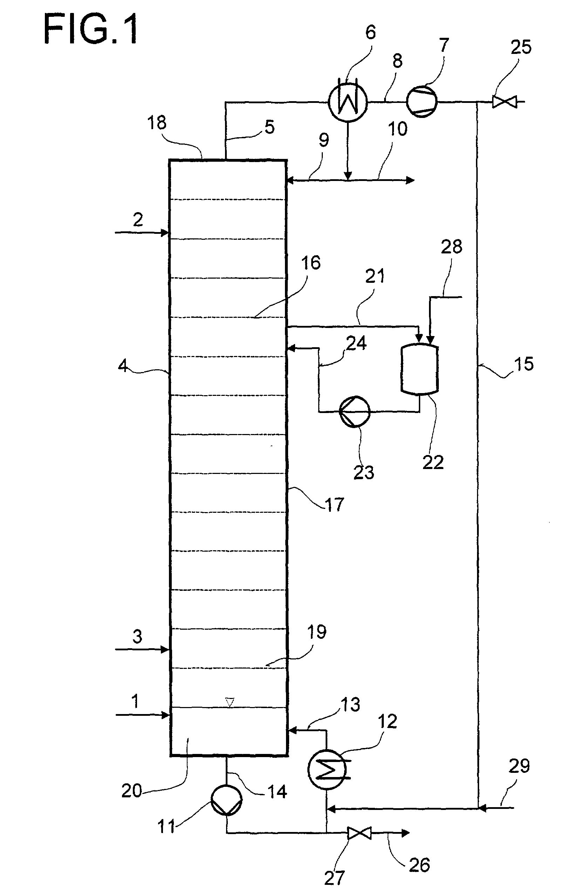

Eine vorteilhafte Durchführung des erfindungsgemäßen Verfahrens wird nachfolgend anhand der Figur 1 beschrieben.

Das erfindungsgemäße Verfahren wird zweckmäßigerweise so durchgeführt, daß man den Alkohol der Formel II, den Acetessigester der allgemeinen Formel III und ggf. die Aluminiumverbindung der allgemeinen Formel V über die Zuläufe (2) und (3) bzw. (1) auf die Einbauten einer Fraktionierkolonne als Reaktionskolonne (4) aufbringt. Dabei ist es von Vorteil, aber nicht zwingend, wenn man den höher siedenden Reaktanten getrennt oder zusammen mit dem Katalysator der Formel V oberhalb des tiefer siedenden Reaktanten kontinuierlich in die Fraktionierkolonne (4) einspeist. Auf den Einbauten der Fraktionierkolonne findet nun die Umsetzung des ungesättigten Alkohols der Formel II mit dem Acetessigester der Formel III zu dem Acetoacetat des Alkohols der Formel II mit einer überlagerten Destillation statt. Dadurch wird der sich aus dem Acetessigsäurealkylester bildende Alkohol der Formel IV ständig aus dem Reaktionsgemisch entfernt, wodurch eine vorteilhafte Gleichgewichtseinstellung erfolgt. Das als Zwischenprodukt gebildete Acetoacetat des Alkohols der Formel II destilliert in den unteren Teil (19) der Fraktionierkolonne (4), wo es bei höheren Temperaturen (von 120 bis 220°C) in Gegenwart der als Katalysator wirkenden Aluminiumverbindung der allgemeinen Formel V in das ungesättigte Keton der Formel I umgelagert und gespalten wird. Das hierbei gebildete Kohlendioxid gelangt über die Fraktionierkolonne (4) in deren Kopfstrom (5).

Die Aluminiumverbindung kann an jeder beliebigen Stelle der Fraktionierkolonne eingespeist werden. Mit besonderem Vorteil bringt man sie aber in den unteren Teil der Kolonne oder gar in den Sumpf ein, da sie erst hier als Katalysator für die Umlagerung und Spaltung des Acetoacetats des Alkohols der Formel II benötigt wird.

Bei Verwendung von Acetessigsäuremethylester oder -ethylester als Ester der Formel III ist es sehr vorteilhaft, wenn man diesen Ester in verdampfter Form in die Kolonne (4) einträgt, da dann die Bildung des Acetoacetats des Alkohols der Formel II schneller erfolgt.

Weiterhin hat es sich als sehr vorteilhaft erwiesen, wenn die Wärmezufuhr in das Reaktorsystem bestehend aus Kolonnensumpf (20) und Fraktionierkolonne (4) und gegebenenfalls Behälter (22) nicht nur über den Verdampfer (12) erfolgt, sondern zusätzlich über außenliegende Wärmetauscher (17) oder über sich direkt auf den Böden (16) befindliche Wärmetauscher erfolgt.

Das gebildete Kohlendioxid und der Alkohol der Formel IV verlassen die Kolonne zusammen mit den Leichtsiedern als Kopfstrom (5) und gelangen in den Kondensator (6), wo die kondensierbaren Bestandteile dieses Brüdenstroms auskondensiert werden. Ein Teil des Kondensats wird als Rücklauf (9) wieder auf die Kolonne gegeben und der andere Teil (10) abgezogen. Es sollte ein Rücklaufverhältnis zwischen 1 und 10, vorzugsweise zwischen 2 und 4, eingestellt werden. Es ist aber auch möglich, das Kondensat vollständig abzuziehen (Rücklaufverhältnis = 0), wenn der höher siedende Reaktant am oberen Ende der Kolonne aufgegeben wird. Das Kohlendioxid verläßt den Kondensator (6) über die Abgasleitung (8). Der Druck am Kolonnenkopf (18) wird so eingestellt, daß die Temperatur im Sumpf (20) zwischen 120 und 300°C, vorzugsweise 160 und 230°C, insbesondere zwischen 170 und 220°C, liegt.
Je nach Stoffsystem und gewünschter Sumpftemperatur kann dies mit einer Vakuumpumpe (7) und/oder einer Druckregeleinrichtung (25) geschehen.

Das Reaktionsprodukt sammelt sich im Sumpf (20) der Kolonne (4) und wird mittels einer Pumpe (11) zusammen mit den nicht umgesetzten Reaktanten über den Sumpfstrom (14) abgezogen. Das Rohprodukt wird mit Hilfe eines Regelventils (27) über die Produktleitung (26) ausgeschleust und der weiteren Aufarbeitung zugeführt. Ein Teil des Sumpfstroms (14) wird in einem Verdampfer (12) verdampft und über die Brüdenleitung (13) wieder in die Kolonne eingespeist. Es ist möglich, zur Entfernung des entstehenden Alkohols der Formel IV zusätzlich ein Inertgas (29) in den Sumpf oder den unteren Teil der Kolonne einzuspeisen und/oder zu diesem Zweck das gebildete, mit dem Kopfstrom ausgeschleuste Kohlendioxid über die Leitung (15) in die Kolonne (4) zurückzuführen.

Die Dosiermengen werden so gewählt, daß das stöchiometrische Verhältnis vom Alkohol der Formel II zu dem Acetessigsäureester der Formel III zwischen 0,8 und 1,2, vorzugsweise zwischen 0,95 und 1,10 liegt und sich ein Katalysatorgehalt von 0,1 bis 5 mol-%, vorzugsweise von 1 bis 3 mol-% bezogen auf den umzusetzenden Acetessigsäurealkylester einstellt. Die Verweilzeit des Reaktionsgemisches im Reaktorsystem bestehend aus Kolonnensumpf (20) und Fraktionierkolonne (4) und gegebenenfalls dem Behälter (22) sollte 15 Minuten bis 6 Stunden, vorzugsweise 1 bis 4 Stunden, betragen. Sie läßt sich vorteilhaft einstellen, wenn man als Kolonneneinbauten Böden (16) mit hoher Verweilzeit der Flüssigkeit verwendet, wie beispielsweise Ventilböden, vorzugsweise Glockenböden oder verwandte Bauarten, wie Tunnelböden.
Es ist aber ebenso möglich, Metallgewebepackungen oder Blechpackungen mit geordneter Struktur oder aber Füllkörperschüttungen als Kolonneneinbauten zu verwenden. Weiterhin ist es ebenfalls möglich, zur Erhöhung der Verweilzeit einen Teilstrom durch einen oder mehrere Seitenabzüge (21) aus der Fraktionierkolonne (4) durch den oder die Behälter (22) zu leiten und mit Hilfe je einer Pumpe (23) die diese Behälter verlassenden Teilströme (24) wieder in die Kolonne (4) zurückzuführen. In die Behälter (22) können mit Hilfe einer Zuleitung (28) gegebenenfalls zusätzlicher Katalysator und/oder Reaktanten zudosiert werden. Eine Beheizung der Behälter (22) ist sinnvoll.

Zur Durchführung der Umsetzung verwendet man mit Vorteil Fraktionierkolonnen, die als Einbauten 10 bis 100 der oben näher beschriebenen Böden (16), vorzugsweise zwischen 20 und 40 Böden, aufweisen. Hierbei arbeitet man mit Vorteil so, daß man den höher siedenden Reaktanten im oberen Teil der Kolonne und den niedriger siedenden Reaktanten im unteren Teil der Kolonne einträgt. Als besonders vorteilhaft hat es sich erwiesen, wenn in der Kolonne 0 bis 5 Böden über dem Zulauf (2) für den höher siedenden Reaktanten und im unteren Teil (19) der Kolonne 0 bis 5 Böden unterhalb des Zulaufs (3) für den niedriger siedenden Reaktanten vorhanden sind. Die Verweilzeit auf den Einbauten der Kolonne sollte zirka 30 Minuten betragen. Entsprechendes gilt für die sogenannten theoretischen Böden bei anderen Kolonneneinbauten.

Bei der Aufarbeitung des Rohproduktstromes (14) aus der Rohproduktleitung (26) ist es von Vorteil, zunächst den Katalysator mit Hilfe eines Verdampfers abzutrennen und anschließend das Wertprodukt in nachfolgenden Destillationskolonnen zu isolieren. Nicht umgesetzte Reaktanten können in die Fraktionierkolonne (4) zurückgeführt werden. Als sehr vorteilhaft bei der Aufarbeitung des Rohproduktstromes erwies sich der Einsatz einer sogenannten Trennwandkolonne.

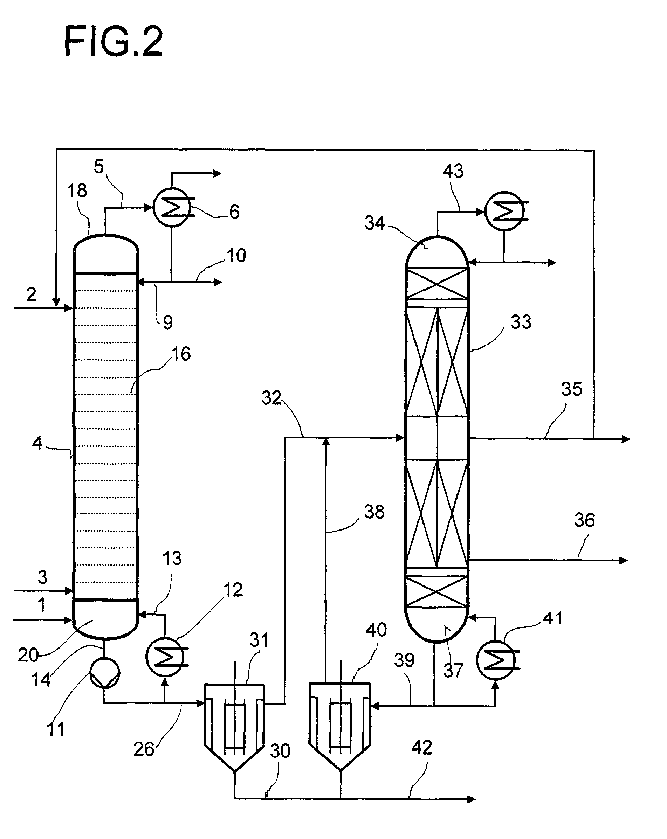

Als Beispiel für die Aufarbeitung des Rohproduktstromes wird im folgenden anhand von Figur 2 die Aufarbeitung unter Verwendung einer Trennwandkolonne näher erläutert.

Bei der Aufarbeitung des Rohproduktstromes (14) aus der Rohproduktleitung (26) ist es von Vorteil, zunächst ein Gemisch (30) aus dem Katalysator und Hochsiedern mit Hilfe eines Verdampfers (31) abzutrennen. Hierdurch wird die Zersetzung von Wertprodukt in den folgenden Aufarbeitungsstufen vermindert. Der abgetrennte Katalysator (30) kann vollständig oder teilweise in die Reaktionskolonne zugeführt werden. Das von Katalysator befreite Gemisch (32) aus Wertprodukt, nicht umgesetzten Reaktanten und leicht siedenden Nebenprodukten wird anschließend in nachgeschalteten Destillationskolonnen aufgetrennt. Für diese Trennaufgabe werden üblicherweise zwei konventionelle Destillationskolonnen benötigt. Bevorzugt ist jedoch der in Figur 2 dargestellte Einsatz einer Trennwandkolonne (33), mit der beispielsweise über deren Kopf (34) der niedriger siedende Reaktant, über einen Seitenabzug (35) der höher siedende Reaktant, über einen weiteren Seitenabzug (36) das ungesättigte Keton der Formel I und über deren Sumpf (37) die Hochsieder abgetrennt werden können. Da mit Hilfe einer Trennwandkolonne die gewünschte Trennung in einer einzigen Apparatur anstatt in zwei Destillationskolonnen möglich ist, werden Investitionskosten eingespart.

Gegenstand der Erfindung ist daher auch das oben genauer beschriebene Verfahren, bei dem man das über den Sumpf (20) der Fraktionierkolonne abgetrennte und über die Produktleitung (26) ausgeschleuste Rohprodukt zur Aufarbeitung entweder direkt oder nach Abtrennen des Gemisches (30) aus dem Katalysator und Hochsiedern mittels eines Verdampfers (31) in einer nachgeschalteten Trennwandkolonne (33) auftrennt.

Der nicht umgesetzte höher siedende Reaktant kann zur Erhöhung der Ausbeuten über den Einlauf (2) in die Reaktionskolonne (4) zurückgeführt werden. Es ist ebenfalls möglich den nicht umgesetzten niedriger siedenden Reaktanten gegebenenfalls nach Ausschleusung eines Teilstromes, in die Reaktionskolonne (4) zurückzuführen. In der Praxis wird jedoch bevorzugt ein vollständiger Umsatz bezüglich des Esters der Formel III angestrebt, so daß der leichtsiedende Nebenkomponenten enthaltende Kopfstrom (43) der Trennwandkolonne üblicherweise vollständig ausgeschleust und entsorgt wird. Der Hochsieder und Wertprodukt enthaltende Sumpfstrom (39) wird in einen zweiten Verdampfer (40) geleitet, in welchem die Hochsieder als Sumpfstrom (42) abgetrennt werden. Der das Wertprodukt enthaltende Strom (38) wird in die Trennwandkolonne (33) zurückgeführt.

In einigen Anwendungfällen des erfindungsgemäßen Verfahrens, wie zum Beispiel bei der Herstellung von 2-Methyl-2-hepten-6-on, siedet der Ester der Formel III höher als der Alkohol der Formel II. In diesem Fall ist es vorteilhafter, den Kopfstrom (43), gegebenenfalls nach Ausschleusung eines Teilstroms, in die Reaktionskolonne (4) zurückzuführen. Die oben geschilderte vollständige Ausschleusung ist in diesem Fall nicht sinnvoll. Der Ester der Formel III kann mit dem Seitenstrom (35) abgezogen und, gegebenenfalls nach Ausschleusung eines Teilstroms, in die Reaktionskolonne (4) zurückgeführt werden.

Zur Einsparung von Investitionskosten ist es auch möglich, anstelle der beiden Verdampfer (31) und (40) nur einen einzigen Verdampfer zu verwenden. Es ist ebenfalls möglich, den Rohproduktstrom (26) direkt in die Trennwandkolonne (33) zu leiten und auf den Verdampfer (31) zu verzichten. In diesem Fall muß darauf geachtet werden, daß die Verweilzeit im Sumpf (37) und Verdampfer (41) der Trennwandkolonne gering gehalten wird, um die Zersetzung von Wertprodukt zu vermeiden. Die Verweilzeit im Sumpf der Trennwandkolonne sollte zweckmäßig weniger als eine Stunde betragen. In diesem Fall wird der Sumpfstrom (39) in dem Verdampfer (40) von Katalysator sowie Hochsiedern befreit und der das Wertprodukt enthaltende Strom (38) in die Trennwandkolonne (33) zurückgeführt. Der im Sumpfstrom (42) abgetrennte Katalysator kann ganz oder teilweise in die Reaktionskolonne (4) zurückgeführt werden.

Mit Hilfe des erfindungsgemäß verbesserten Verfahrens ist es möglich, zahlreiche höhere Ketone, insbesondere solche Ketone, wie sie zur Herstellung von Isophytol und damit zur Herstellung von Vitamin E benötigt werden, wie Geranylaceton, Farnesylaceton, 6,10-Dimethyl-5-undecen-2-on, 6,10,14-Trimethyl-5,10-pentadecadien-2-on und 2-Methyl-2-hepten-6-on, bei nahezu quantitativem Umsatz in sehr hohen Ausbeuten und Raumzeitausbeuten sowie hoher Reinheit herzustellen. Mit Hilfe des erfindungsgemäßen Verfahrens ist es auch möglich, daß man ein Gemisch aus zwei verschiedenen ungesättigten Alkoholen der allgemeinen Formel II mit einem Acetessigsäurealkylester der allgemeinen Formel III zu einem Gemisch der entsprechenden ungesättigten Ketone der allgemeinen Formel I umsetzt und dieses Gemisch anschließend destillativ aufarbeitet. Dies ist z.B. von Vorteil, wenn man wechselnde Mengen an verschiedenen ungesättigten Ketonen herstellen möchte, die benötigten Ketonmengen aber nicht so groß sind, daß für jedes Keton eine eigene Produktionsanlage gebaut werden muß.

### Beispiel 1

### Herstellung von 6,10-Dimethyl-5-undecen-2-on (Dihydrogeranylaceton)

A. Beschreibung der Apparatur
   Als Apparatur wurde eine Fraktionierkolonne (4) mit 30 Glockenböden (ca. 20 theoretische Stufen) und einem Innendurchmesser von 30 mm verwendet. Die Numerierung der Böden erfolgte von unten nach oben, d.h. der unterste Boden war Boden 1 und der oberste Boden war Boden 30. Die Kolonne war in regelmäßigen Abständen mit Thermoelementen bestückt, so daß außer am Sumpf (20) und am Kopf (18) der Kolonne an jeder 3. bis 4. theoretischen Stufe die Temperatur gemessen werden konnte. Zusätzlich zum Temperaturprofil konnte mit Hilfe entsprechender Probenentnahmestellen das Konzentrationsprofil in der Kolonne ermittelt werden. Der Verdampfer (12), der mit Hilfe eines Thermostaten auf 250°C beheizt wurde, hatte ein Volumen von ca. 350 ml, wobei der Füllstand während des Betriebs etwa 225 ml betrug. Auf die Kolonne war ein Kühler (6) aufgesetzt, der mit einem Kryostaten betrieben wurde. Weiterhin war die Kolonne mit einem Vakuum-Aggregat (7) und einer Kühlfalle ausgestattet. Alle zu- und ablaufenden Ströme wurden mit Hilfe von Waagen erfaßt und aufgezeichnet.
B. Versuchsdurchführung - Herstellung von 6,10-Dimethyl-5-undecen-2-on
   Auf den Boden 27 der Kolonne (4) wurden 135,0 g/h (0,81 mol/h) 3,7-Dimethyl-1-octen-3-ol (Dihydrolinalool, 94%ig) und auf den Boden 3 der Kolonne 94,0 g/h (0,81 mol/h) Acetessigsäuremethylester (AME) aufgebracht. Als Katalysator wurde ein durch Umsetzung von Aluminium-sek.-butylat mit AME hergestelltes, gemischtes Aluminiumtriacetoacetat in Form einer methanolischen Lösung eingesetzt. Der Katalysator wurde durch Elementaranalyse und ¹H-NMR charakterisiert, wobei sich ein Aluminiumgehalt von 5,0 Gew.-% und ein Umesterungsgrad (Methanol gegen 2-Butanol) von 50 % ergab. 7,6 g/h (1,5 mol-% Aluminium bezogen auf eingesetzten AME) dieses Katalysators wurden zusammen mit dem 3,7-Dimethyl-1-octen-3-ol auf den Boden 27 dosiert. Es wurde ein Systemdruck von 500 mbar und ein Rücklaufverhältnis von 3 eingestellt. Die Sumpftemperatur betrug 200°C und die Verweilzeit in dem Reaktorsystem 2 Stunden. Als Sumpfstrom wurden 171,7 g/h Rohprodukt enthaltend 87,4 Gew.-% 6,10-Dimethyl-5-undecen-2-on, 3,8 Gew.-% 3,7-Dimethyl-1-octen-3-ol, 0,4 Gew.-% AME und 8,4 Gew.-% Hochsieder gewonnen. Am Kopf (18) der Kolonne wurden 23,3 g/h Destillat, enthaltend 89,5 Gew.-% Methanol, abgezogen. Das während der Reaktion entstandene Kohlendioxid wurde über den Kopf (18) der Kolonne abgeleitet. Das 6,10-Dimethyl-5-undecen-2-on wurde mit einer Selektivität von 99,2 %, bezogen auf 3,7-Dimethyl-1-octen-3-ol und 95,4 % bezogen auf AME erhalten. Der Umsatz betrug 95 %, bezogen auf 3,7-Dimethyl-1-octen-3-ol, und 99,2 %, bezogen auf AME. Aus dem Rohprodukt wurde in einer Destillationskolonne mit 22 theoretischen Stufen 6,10-Dimethyl-5-undecen-2-on in einer Reinheit von 99,99 % erhalten.

### Beispiel 2

### Herstellung von 6,10,14-Trimethyl-5,10-pentadecadien-2-on (Dihydrofarnesylaceton)

A. Erfindungsgemäße kontinuierliche Herstellung.
   In der in Beispiel 1 beschriebenen Apparatur wurden auf den Boden 27 der Kolonne 152,0 g/h (0,63 mol/h) 3,7,11-Trimethyl-1,6-dodecadien-3-ol (Dihydronerolidol, 93%ig) und auf den Boden 3 der Kolonne 73,0 g/h (0,63 mol/h) AME aufgebracht. Als Katalysator wurde ein durch Umsetzung von Aluminium-sek.-butylat mit AME hergestelltes, gemischtes Aluminiumtriacetoacetat als 80 %ige methanolische Lösung eingesetzt. Der Katalysator wurde durch Elementaranalyse und ¹H-NMR charakterisiert, wobei sich ein Aluminiumgehalt von 5,4 Gew.-% und ein Umesterungsgrad (Methanol gegen 2-Butanol) von 50 % ergab. 4,9 g/h (1,5 mol-% Aluminium bezogen auf den AME) dieses Katalysators wurden zusammen mit dem 3,7,11-Trimethyl-1,6-dodecadien-3-ol auf den Boden 27 dosiert. Es wurde ein Systemdruck von 500 mbar und ein Rücklaufverhältnis von 3 eingestellt. Die Sumpftemperatur betrug 200°C und die Verweilzeit in dem Reaktorsystem 2 Stunden. Als Sumpfstrom wurden 180,4 g/h Rohprodukt enthaltend 82,3 Gew.-% 6,10,14-Trimethyl-5,10-pentadecadien-2-on, 5,3 Gew.-% 3,7,11-Trimethyl-1,6-dodecadien-3-ol, 1,1 Gew.-% AME und 11,3 Gew.-% Hochsieder gewonnen. Am Kopf der Kolonne wurden 14,6 g/h Destillat bestehend aus 88,1 Gew.-% Methanol abgezogen. Das während der Reaktion entstandene Kohlendioxid wurde über den Kopf der Kolonne abgeleitet. Das 6,10,14-Trimethyl-5,10-pentadecadien-2-on wurde mit einer Selektivität von 95,6 % bezogen auf 3,7,11-Trimethyl-1,6-dodecadien-3-ol und 91,7 % bezogen auf AME erhalten. Der Umsatz betrug 93,5 % bezogen auf 3,7,11-Trimethyl-1,6-dodecadien-3-ol und 97,4 Gew.-% bezogen auf AME.
B. Diskontinuierlicher Vergleichsversuch
   Die Versuchsapparatur bestand aus einem beheizbaren, mit Rührer ausgestatteten 2-Liter-Reaktionskolben aus Edelstahl, auf den eine Destillationskolonne (Länge: 1 m, Durchmesser: 25 mm) aufgesetzt war. Die Kolonne war mit Edelstahldrahtwendeln (5 mm) gefüllt. Die Reaktanten wurden entweder in der Reaktionsblase vorgelegt oder mit Hilfe einer Pumpe zudosiert. Die während der Reaktion freigesetzten Komponenten, Methanol und CO₂, wurden über die Kolonne abgetrennt und kondensiert. Alle ein- und austretenden Stoffströme wurden während des gesamten Versuchs kontinuierlich erfaßt und registriert, so daß eine zeitabhängige Massenbilanzierung möglich war.
   657,6 g (2,73 mol) 3,7,11-Trimethyl-1,6-dodecadien-3-ol (Dihydronerolidol, 93%ig) wurden im Reaktionskolben mit aufgesetzter Kolonne vorgelegt und auf 100°C erhitzt. Anschließend wurden 27,7 g (1,5 mol-% bezogen auf AME) der oben beschriebenen Katalysatorlösung mit 35,4 g (0,15 mol) 3,7,11-Trimethyl-1,6-dodecadien-3-ol und 7,5 g (0,06 mol) AME vermischt, die Mischung dem Kolbeninhalt zugesetzt und die Reaktionsmischung auf 175°C aufgeheizt. Danach wurden 326,5 g (2,82 mol) AME innerhalb von 3,5 h in die Reaktionsblase dosiert. Während der AME-Zugabe wurde die Reaktionslösung weiter aufgeheizt und die Temperatur bei 185°C eingeregelt. Mit Beginn der AME-Zugabe setzte die CO₂-Entwicklung ein, und das gebildete Methanol wurde als Destillat abgenommen. Das Rücklaufverhältnis betrug 0,1. Nach ca. 3,5 h war die AME-Dosierung abgeschlossen. Mit Abschluß der AME-Dosierung begann die Nachreaktion. Auch während der Nachreaktion wurde die Temperatur bei 185°C konstant gehalten. Die Nachreaktion war nach 2 h beendet. Das 6,10,14-Trimethyl-5,10-pentadecadien-2-on wurde so mit einer Selektivität von 93,9 % bezogen auf 3,7,11-Trimethyl-1,6-dodecadien-3-ol und 91,8 bezogen auf AME erhalten.

### Beispiel 3

### Bevorzugte Fahrweise für die Herstellung von Dihydrofarnesylaceton

In der in Beispiel 1 beschriebenen Apparatur wurden auf den Boden 27 der Kolonne (4) 155,0 g/h (0,64 mol/h) 3,7,11-Trimethyl-1,6-dodecadien-3-ol (Dihydronerolidol, 92 %ig) gepumpt. Im Unterschied zu Beispiel 2 wurden 74,0 g/h (0,64 mol/h) AME dampfförmig in den Dampfraum oberhalb des Sumpfes (20) der Kolonne (4) dosiert. Als Katalysator wurde ein gemischtes Aluminium-triacetoacetat eingesetzt, das durch diskontinuierliche Umsetzung von 500 g (2,03 mol) Aluminium-tri-sek.-butylat mit 707 g (6,09 mol) AME hergestellt worden war. Der Katalysator, der - bedingt durch die Herstellung als Lösung - mit ca. 18 Gew.-% 2-Butanol und 6 Gew.-% Methanol eingesetzt wurde, wurde durch Elementaranalyse und ¹H-NMR charakterisiert. Hierbei ergab sich ein Aluminiumgehalt von 4,4 Gew.-% und ein Umesterungsgrad (Methanol gegen 2-Butanol) von ca. 50 %. 6 g/h der Katalysatorlösung (1,5 Mol-% Aluminium bezogen auf AME) wurden im Unterschied zu Beispiel 1 direkt in den Sumpf (20) gepumpt. Es wurde ein Systemdruck von 500 mbar und ein Rücklaufverhältnis von 3 eingestellt. Die Temperatur im Sumpf (20) betrug 200°C und die Verweilzeit in dem Reaktorsystem 2 Stunden. Als Sumpfstrom (14) wurden 182,7 g/h Rohprodukt enthaltend 82,7 Gew.-% 6,10,14-Trimethyl-5,10-pentadecadien-2-on, 4,9 Gew.-% 3,7,11-Trimethyl-1,6-dodecadien-3-ol, 1,0 Gew.-% AME 11,4 Gew.-% Hochsieder gewonnen. Am Kopf (18) der Kolonne wurden 16,7 g/h Destillat bestehend aus 80,6 Gew.-% Methanol und 8,6 Gew.-% 2-Butanol als Kopfstrom (5) abgezogen. Auch das während der Reaktion entstandene Kohlendioxid wurde über den Kopf (18) der Kolonne (4) abgeleitet. Das 6,10,14-Trimethyl-5,10-pentadecadien-2-on wurde mit einer Selektivität von 96,0 %, bezogen auf 3,7,11-Trimethyl-1,6-dodecadien-3-ol und 91,8 %, bezogen auf AME, erhalten. Der Umsatz betrug 93,7 %, bezogen auf 3,7,11-Trimethyl-1,6-dodecadien-3-ol, und 97,7 %, bezogen auf AME. Das Rohprodukt wurde in einer nachfolgenden Trennwandkolonne (33) mit 30 theoretischen Stufen aufgearbeitet, wobei über den Seitenabzug (36) das 6,10,14-Trimethyl-5,10-pentadecadien-2-on mit einer Reinheit von 99,99 % und über den Seitenabzug (36) das nicht umgesetzte 3,7,11-Trimethyl-1,6-dodecadien-3-ol mit einer Reinheit von 90 % abgezogen wurde.

### Beispiel 4

### Kontinuierliche gemeinsame Produktion von Dihydrogeranylaceton und Dihydrofarnesylaceton

In der unter Beispiel 1 beschriebenen Apparatur wurden auf den Boden 27 der Kolonne (4) 84,0 g/h (0,35 mol/h) 3,7,11-Trimethyl-1,6dodecadien-3-ol (Dihydronerolidol, 92%ig) und 58,5 g/h (0,34 mol/h) 3,7-Dimethyl-1-octen-3-ol (Dihydrolinalool, 90,5 %ig) gepumpt. Auf den Boden 3 der Kolonne (4) wurden 80,0 g/h (0,69 mol/h) AME dosiert. Als Katalysator wurde ein gemischtes Aluminiumtriacetoacetat eingesetzt, das durch diskontinuierliche Umsetzung von 500 g (2,03 mol) Aluminium-tri-sek.-butylat mit 707 g (6,09 mol) AME ohne Entfernen des freigesetzten 2-Butanols hergestellt worden war. Der Katalysator, der - bedingt durch die Herstellung als Lösung - enthaltend ca. 18 Gew-% 2-Butanol und 6 Gew.-% Methanol eingesetzt wurde, wurde durch Elementaranalyse und ¹H-NMR charakterisiert. Hierbei ergab sich ein Aluminiumgehalt von 4,4 Gew.-% und ein Umesterungsgrad (Methanol gegen 2-Butanol) von ca. 50 %. 6,5 g/h der Katalysatorlösung (1,5 Mol-% Aluminium, bezogen auf AME) wurden auf den Boden 27 der Kolonne (4) gepumpt. Es wurde ein Systemdruck von 500 mbar und ein Rücklaufverhältnis von 3 eingestellt. Die Temperatur im Sumpf (20) betrug 200°C und die Verweilzeit in dem Reaktorsystem 2 Stunden. Als Sumpfstrom (14) wurden 172,0 g/h Rohprodukt enthaltend 46,9 Gew.-% 6,10,14-Trimethyl-5,10-pentadecadien-2-on, 36,1 Gew.-% 6,10-Dimethyl-5-undecen-2-on, 2,7 Gew.-% 3,7,11-Trimethyl-1,6-dodecadien-3-ol, 1,6 Gew.-% 3,7-Dimethyl-1-octen-3-ol, 0,9 Gew.-% AME und 13,4 Gew.-% Hochsieder gewonnen. Am Kopf (18) der Kolonne (4) wurden 20,2 g/h Destillat bestehend aus 80,9 Gew.-% Methanol und 11,3 Gew.-% 2-Butanol als Kopfstrom (5) abgezogen. Auch das während der Reaktion entstandene Kohlendioxid wurde über den Kopf (18) der Kolonne abgeleitet. Das 6,10,14-Trimethyl-5,10-pentadecadien-2-on wurde mit einer Selektivität von 94,0 %, bezogen auf 3,7,11-Trimethyl-1,6-dodecadien-3-ol, und das 6,10-Dimethyl-5-undecen-2-on mit einer Selektivität von 98,4 %, bezogen auf 3,7-Dimethyl-l-octen-3-ol, erhalten. Die Selektivität der Wertprodukte, bezogen auf AME betrug 91,9 %. Der Umsatz betrug 94,1 %, bezogen auf 3,7,11-Trimethyl-1,6-dodecadien-3-ol, 94,7 %, bezogen auf 3,7-Dimethyl-1-octen-3-ol und 98,2 %, bezogen auf AME.

### Beispiel 5

### Herstellung von Dihydrofarnesylaceton in einer Reaktionskolonne mit strukturierter Packung

A. Beschreibung der Apparatur
   Als Reaktionsapparatur wurde eine Destillationskolonne (4) mit einem Innendurchmesser von 30 mm verwendet. Der untere Teil der Kolonne war bis zu einer Höhe von einem Meter mit einer strukturierten Blechpackung (Sulzer Mellapak 250.Y) gepackt. Oberhalb dieser Blechpackung befanden sich 0,5 Meter einer strukturierten Metall-Gewebepackung (Sulzer DX). Die Kolonne (4) war in regelmäßigen Abständen mit Thermoelementen bestückt, so daß außer am Sumpf (20) und am Kopf (18) der Kolonne an mehreren Stellen in der Kolonne die Temperatur gemessen werden konnte. Zusätzlich zum Temperaturprofil wurde mit Hilfe entsprechender Probennahmestellen das Konzentrationsprofil in der Kolonne (4) ermittelt. Der Verdampfer, der mit Hilfe eines Thermostaten auf 250°C beheizt wurde, hatte ein Volumen von ca. 350 ml, wobei der Füllstand während des Betriebs etwa 225 ml betrug. Auf die Kolonne (4) wurde ein Kühler (6) aufgesetzt, der mit einem Kryostaten betrieben wurde. Weiterhin war die Kolonne (4) mit einem Vakuumaggregat (7) und einer Kühlfalle ausgestattet. Alle zu- und ablaufenden Ströme wurden mit Hilfe von Waagen erfaßt und aufgezeichnet.
B. Versuchsdurchführung
   In der oben beschriebenen Apparatur wurden 155,0 g/h (0,64 mol/h) 3,7,11-Trimethyl-1,6-dodecadien-3-ol (Dihydronerolidol, 92 %ig) auf das obere Packungssegment (DX-Packung) gepumpt. 74,0 g/h (0,64 mol/h) AME wurden dampfförmig in den Dampfraum oberhalb des Sumpfes (20) dosiert. Als Katalysator wurde das in Beispiel 4 beschriebene gemischte Aluminiumtriacetoacetat eingesetzt. 6 g/h dieser Katalysatorlösung (1,5 Mol-% Aluminium, bezogen auf AME) wurden 10 cm oberhalb des Sumpfes (20) in die Kolonne gepumpt. Es wurde ein Systemdruck von 500 mbar und ein Rücklaufverhältnis von 3 eingestellt. Die Sumpftemperatur betrug 200°C und die Verweilzeit in dem Reaktorsystem 2 Stunden. Als Sumpfstrom (14) wurden 184,9 g/h Rohprodukt enthaltend 80,8 Gew.-% 6,10,14-Trimethyl-5,10-pentadecadien-2-on, 6,1 Gew.-% 3,7,11-Trimethyl-1,6-dodecadien-3-ol, 0,9 Gew.-% AME und 12,2 Gew.-% Hochsieder gewonnen. Am Kopf (18) der Kolonne wurden 16,0 g/h Destillat bestehend aus 83,0 Gew.-% Methanol und 4,2 Gew.-% 2-Butanol abgezogen. Auch das während der Reaktion entstandene Kohlendioxid wurde über den Kopf (18) abgeleitet. Das 6,10,14-Trimethyl-5,10-pentadecadien-2-on wurde mit einer Selektivität von 96,5 %, bezogen auf 3,7,11-Trimethyl-1,6-dodecadien-3-ol und 90,8 %, bezogen auf AME erhalten. Der Umsatz betrug 92,1 %, bezogen auf 3,7,11-Trimethyl-1,6-dodecadien-3-ol, und 97,6 Gew.-%, bezogen auf AME.

### Beispiel 6

### Herstellung von 2-Methyl-2-hepten-6-on

### Erfindungsgemäße kontinuierliche Herstellung

A. Beschreibung der Versuchsapparatur
   Als Apparatur wurde eine unter Druck betriebene Reaktionskolonne verwendet. Diese Reaktionskolonne bestand aus einem beheizbaren, mit Rührer ausgestatteten 2-Liter-Reaktionskolben aus Edelstahl mit aufgesetzter Destillationskolonne (Länge: 1,2 m, Durchmesser: 54 mm). Als Kolonneneinbauten wurden strukturierte Gewebepackungen vom Typ Montz A3-750 eingesetzt und somit etwa 14 theoretische Stufen realisiert. Der AME wurde bei Raumtemperatur kontinuierlich in den oberen Teil der Reaktionskolonne gegeben, 2-Methyl-3-buten-2-ol in den Dampfraum der Reaktionsblase geflasht und der Katalysator flüssig in die Reaktionsblase dosiert. Die während der Reaktion freigesetzten Komponenten Methanol und CO₂ sowie die flüchtigen Nebenprodukte Isopren und Aceton wurden über die Kolonne abgetrennt. Die Leichtsieder des am Kolonnenkopf anfallenden Brüdenstroms wurden in einem Teilkondensator kondensiert, wobei ein Teil des Kondensats über einen Rücklaufteiler in einen Vorlagebehälter lief, während der andere Teil als Rücklauf auf die Kolonne gegeben wurde. Der verbleibende, hauptsächlich aus CO₂ bestehende Abgasstrom wurde durch eine Kühlfalle und anschließend zur Volumenmessung durch eine Gasuhr geleitet. Das Reaktionsprodukt wurde dem Kolonnensumpf (2-Liter-Reaktionskolben) mittels einer Standregelung kontinuierlich entnommen. Der Katalysator und die Hochsieder wurden durch einstufige Destillation vom Sumpfprodukt abgetrennt und das Destillat gaschromatographisch analysiert. Ebenso wurde das Destillat der Reaktionskolonne gaschromatographisch analysiert. Die Apparatur war mit einer Druckregelung ausgestattet und auf einen Systemdruck von 10 bar ausgelegt. Alle ein- und austretenden Stoffströme wurden während des gesamten Versuchs kontinuierlich erfaßt und registriert, so daß eine zeitabhängige Massenbilanzierung möglich war.
B. Versuchsdurchführung
   81,9 g/h (0,70 mol/h) AME wurden kontinuierlich auf die Stufe 3 der Kolonne dosiert (Zählung der Stufen erfolgt von oben nach unten, wobei der Kondensator Stufe 0 entspricht). In den Dampfraum der Reaktionsblase wurden 67,0 g/h 2-Methyl-3-buten-2-ol (MBE; 92%ig; 0,72 mol/h berechnet auf 100 %) geflasht. Als Katalysator wurde ein durch Umsetzung von Aluminium-sek.-butylat mit Acetessigsäuremethylester hergestelltes, gemischtes Aluminiumtriacetoacetat eingesetzt. Der Katalysator wurde durch Elementaranalyse und ¹H-NMR charakterisiert, wobei sich ein Aluminiumgehalt von 4,6 Gew.-% und ein Umesterungsgrad (Methanol gegen 2-Butanol) von 50 % ergab. 8,2 g/h (2,0 Mol-% Aluminium, bezogen auf AME) dieses Katalysators wurden in die Reaktionsblase dosiert. Es wurde ein Systemdruck von 1,5 bar und ein Rücklaufverhältnis von 5 kg/kg eingestellt. Die Sumpftemperatur betrug 171°C und die Verweilzeit in dem Reaktorsystem 3,7 Stunden. Als Sumpfstrom wurden 99,6 g/h Rohprodukt mit 82,4 Gew.-% 2-Methyl-2-hepten-6-on, 3,8 Gew.-% 2-Methyl-3-buten-2-ol, 1,2 Gew.-% Acetessigsäuremethylester, 0,3 Gew.-% Methanol und 12,0 Gew.-% Hochsieder gewonnen. Der Gehalt des unerwünschten Nebenprodukts 2-Methyl-2-hepten-6-ol lag unter 0,2 Gew.-%. Am Kopf der Kolonne wurden 23,7 g/h Destillat bestehend aus 85,5 Gew.-% Methanol abgezogen. Das während der Reaktion entstandene Kohlendioxid wurde über Kopf abgeleitet. Es wurde 2-Methyl-2-hepten-6-on mit einer Selektivität von 97,7 %, bezogen auf 2-Methyl-3-buten-2-ol, und 94,5 %, bezogen Acetessigsäuremethylester erhalten. Die Ausbeute betrug 90,8 %, bezogen auf 2-Methyl-3-buten-2-ol, und 93,1 Gew.-%, bezogen auf AME.
   Aus dem Rohprodukt wurde nach Abtrennung der Hochsieder mittels eines Dünnschichtverdampfers und Rektifikation in einer Rektifikationskolonne mit 30 theoretischen Stufen 2-Methyl-2-hepten-6-on in einer Reinheit von 99,8 % erhalten.

### Beispiel 7

### Herstellung von 2-Methyl-2-hepten-6-on

### Diskontinuierlicher Vergleichsversuch

A. Beschreibung der Versuchsapparatur
   Die Versuchsapparatur bestand aus einem beheizbaren, mit Rührer ausgestatteten 2-Liter-Reaktionskolben aus Edelstahl, auf den eine Destillationskolonne (Länge: Im; Durchmesser: 25 mm) aufgesetzt war. Die Kolonne war mit Edelstahldrahtwendeln (Durchmesser: 5 mm) gefüllt. Die Reaktanten können entweder in der Reaktionsblase vorgelegt oder mit Hilfe einer Pumpe aus einem Vorlagebehälter, der auf einer Waage steht, in die Reaktionsblase dosiert werden. Die während der Reaktion freigesetzten Komponenten Methanol und CO₂, sowie die flüchtigen Nebenprodukte Isopren und Aceton wurden über die Destillationskolonne abgetrennt und die Leichtsieder in einem Teilkondensator kondensiert. Das Kondensat lief über einen Rücklaufteiler in einen Vorlagebehälter. Der verbleibende, hauptsächlich aus CO₂ bestehende, Abgasstrom wurde durch eine Kühlfalle und anschließend zur Volumenmessung durch eine Gasuhr geleitet. Die Apparatur war mit einer Druckregelungsvorrichtung ausgestattet und auf einen Systemdruck von 10 bar ausgelegt. Alle ein- und austretenden Stoffströme wurden während des gesamten Versuchs kontinuierlich erfaßt und registriert, so daß eine zeitabhängige Massenbilanzierung möglich war.
B. Versuchsdurchführung
   Es wurden 680,0 g 2-Methyl-3-buten-2-ol (MBE; 93%ig; 7,34 mol berechnet auf 100 %) und 96,7 g Katalysator (1,75 Mol-% Aluminium, bezogen auf AME) in dem 2-Liter-Rührautoklaven mit aufgesetzter Kolonne vorgelegt. Als Katalysator wurde analog zu Beispiel 6 ein durch Umsetzung von Aluminium-sek.-butylat mit AME hergestelltes, gemischtes Aluminiumtriacetoacetat eingesetzt. Anschließend wurde mit einer Gasflasche Stickstoff aufgepreßt und ein Reaktordruck von 1,7 bar (absolut) eingestellt. Danach wurde das Gemisch aus MBE und Katalysator bei unendlichem Rücklaufverhältnis mit dem Thermostaten auf 130°C aufgeheizt. Ab einer Temperatur von 130°C wurden 811,3 g (6,99 mol) AME innerhalb von 180 Minuten (min) linear in den Rührautoklaven eindosiert. Mit dem Beginn der Dosierung sprang die Reaktion an, so daß die CO₂-Entwicklung einsetzte. Hierdurch wurde innerhalb von 30 min ein Reaktionsdruck von 4 bar (absolut) aufgebaut. Der Druck wurde anschließend über die Druckregelungsvorrichtung aufrechterhalten.
   In der Anfahrphase destillierte überwiegend MBE in die Kolonne, das bei totalem Rücklauf in den Rührautoklaven zurückgeführt wurde. Das MBE wurde mit Beginn der Dosierung durch das während der Reaktion freigesetzte Methanol verdrängt. Sobald eine Kopftemperatur von 104°C (Siedetemperatur von Methanol bei 4 bar) erreicht war, wurde das Rücklaufverhältnis (RV) auf 14 eingestellt. Das entstandene Methanol wurde über die aufgesetzte Kolonne als Destillat abgetrennt und das CO₂ mit Hilfe der Druckregelungsvorrichtung so abgeführt, daß der Reaktordruck auf 4 bar gehalten wurde. Die Temperatur (T) des Reaktionsgemisches stieg während der Reaktion auf 185°C an und wurde danach auf 185°C geregelt. Nach 180 min war die AME-Dosierung abgeschlossen. Mit Abschluß der AME-Dosierung begann die Nachreaktion. Die Apparatur wurde langsam von 4 bar auf Normaldruck entspannt, wobei die Temperatur (T) von 185°C auf 170°C absank. Die T wurde anschließend auf 170°C geregelt. Die Nachreaktion wurde nach 90 min beendet. Der Katalysator und die Hochsieder wurden durch einstufige Destillation abgetrennt und das Destillat gaschromatographisch analysiert.
   2-Methyl-2-hepten-6-on wurde mit einer Selektivität von 93,4 %, bezogen auf 2-Methyl-3-buten-2-ol, und 89,0 %, bezogen auf AME, erhalten. Die Ausbeute betrug 85,6 %, bezogen 2-Methyl-3-buten-2-ol, und 89,0 Gew.-%, bezogen auf AME.

## Patentansprüche

1. Verfahren zur kontinuierlichen Herstellung von ungesättigten Ketonen der allgemeinen Formel I 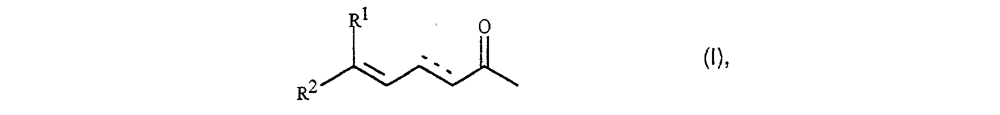 in der die gestrichelte Linie eine zusätzliche C-C-Bindung bedeuten kann, R¹ für eine verzweigte oder unverzweigte Alkylgruppe mit 1 bis 4 C-Atomen steht, und R² für einen gesättigten oder ungesättigten, verzweigten oder unverzweigten, gegebenenfalls durch Methoxygruppen substituierten aliphatischen Kohlenwasserstoffrest mit 1 bis 37 C-Atomen, eine Cycloalkylgruppe mit 4 bis 12 C-Atomen oder eine Cycloalkylalkylgruppe mit 5 bis 30 C-Atomen steht, durch Umsetzen eines ungesättigten Alkohols der allgemeinen Formel II 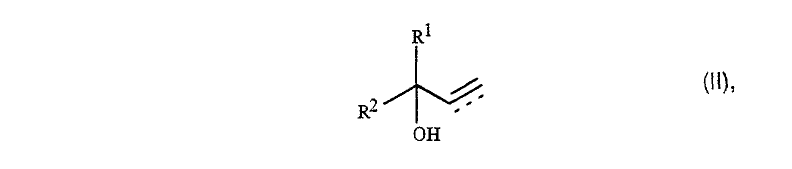 in der R¹ und R² die obenangegebene Bedeutung haben,
mit einem Acetessigsäurealkylester der allgemeinen Formel III 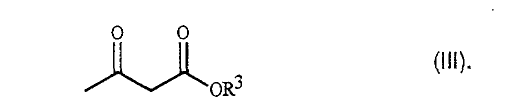 in der R³ für eine verzweigte oder unverzweigte Alkylgruppe mit 1 bis 5 C-Atomen steht, in Gegenwart organischer Aluminiumverbindungen als Katalysator
**dadurch gekennzeichnet, daß** man
A. den ungesättigten Alkohol der Formel II mit dem Acetessigsäurealkylester der Formel III auf den Einbauten (16) einer Fraktionierkolonne (4) zu dem Acetoacetat des ungesättigten Alkohols umsetzt und den dabei entstehenden Alkohol der allgemeinen Formel IV
R³-OH (IV)
mit dem Kopfstrom (5) der Fraktionierkolonne abtrennt und
B. das gebildete Acetoacetat des Alkohols der Formel II im unteren Teil der Fraktionierkolonne und/oder im Sumpf (20) der Fraktionierkolonne in Gegenwart der organischen Aluminiumverbindung in das ungesättigte Keton der Formel I umlagert, wobei das sich hierbei bildende Kohlendioxid über die Fraktionierkolonne (4) in den Kopfstrom (5) gelangt und das gebildete ungesättigte Keton der allgemeinen Formel I über den Sumpf (20) der Fraktionierkolonne abgetrennt wird.

2. Verfahren gemäß Anspruch 1, **dadurch gekennzeichnet, daß** die Stufe B. der Umsetzung in Gegenwart von 0,1 bis 5 Mol-%, bezogen auf den umzusetzenden Acetessigsäurealkylester, einer organischen Aluminiumverbindung der allgemeinen Formel V 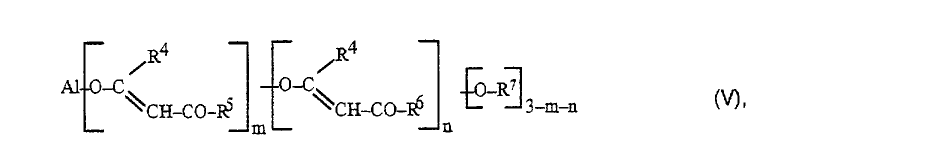 in der R⁴ verzweigte oder unverzweigte Alkyl- oder Alkoxygruppen mit 1 bis 4 C-Atomen, vorzugsweise Methyl- oder Ethylgruppen bedeuten, R⁵ und R⁶ verzweigte oder unverzweigte Alkyl- oder Alkoxygruppen mit 1 bis 5 C-Atomen, vorzugsweise eine Methyl- oder eine 2-Butyl-gruppe bedeutet, R⁷ für eine verzweigte oder unverzweigte Alkylgruppe mit 1 bis 4 C-Atomen steht und m sowie n eine ganze Zahl von 0 bis 3 bedeuten können, wobei n+m ≤ 3 ist, oder aber in Gegenwart von Aluminiumtriaryloxylaten als Katalysator durchgeführt wird.

3. Verfahren gemäß Anspruch 1, **dadurch gekennzeichnet, daß** man als ungesättigten Alkohol der allgemeinen Formel II einen solchen verwendet, in dem R¹ für CH₃ und R² für eine Gruppe der allgemeinen Formel VI 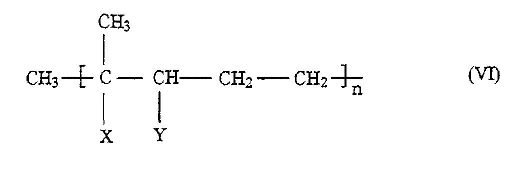 steht, in der n für eine ganze Zahl von 1 bis 6 steht und x und y entweder beide für H stehen, oder x für Methoxy und y für H stehen, oder aber x und y zusammen eine zusätzliche Bindung zwischen den X- und Y-tragenden C-Atomen bedeuten.

4. Verfahren gemäß Anspruch 1, **dadurch gekennzeichnet, daß** man die Einsatzmengen so wählt, daß sich ein Molverhältnis von Alkohol der Formel II zu Acetessigsäureester der Formel III zwischen 0,8 und 1,2 ergibt.

5. Verfahren gemäß Anspruch 1, **dadurch gekennzeichnet, daß** man den höher siedenden Reaktanten oberhalb des tiefer siedenden Reaktanten in die Fraktionierkolonne (4) einspeist.

6. Verfahren gemäß Anspruch 1, **dadurch gekennzeichnet, daß** man den Katalysator in flüssiger Form in den Sumpf (20) der Fraktionierkolonne (4) einspeist.

7. Verfahren gemäß Anspruch 1, **dadurch gekennzeichnet, daß** der Druck am Kolonnenkopf so eingestellt wird, daß die Temperatur im Sumpf (20) zwischen 120 und 300°C liegt.

8. Verfahren gemäß Anspruch 1, **dadurch gekennzeichnet, daß** die Verweilzeit im Reaktorsystem bestehend aus Kolonnensumpf (20) und Fraktionierkolonne (4) und gegebenenfalls Behälter (22) zwischen 15 Minuten und 6 Stunden liegt.

9. Verfahren gemäß Anspruch 1, **dadurch gekennzeichnet, daß** man als Einbauten für die Fraktionierkolonne (4) Kolonnenböden verwendet, wobei die Böden (16) eine hohe Verweilzeit der Flüssigkeit ermöglichen sollen.

10. Verfahren gemäß Anspruch 1, **dadurch gekennzeichnet, daß** die Wärmezufuhr in das Reaktorsystem bestehend aus Kolonnensumpf (20) und Fraktionierkolonne (4) und gegebenenfalls Behälter (22) nicht nur über den Verdampfer (12) erfolgt, sondern zusätzlich über außenliegende Wärmetauscher (17) oder über sich direkt auf den Böden (16) befindliche Wärmetauscher erfolgt.

11. Verfahren gemäß Anspruch 1, **dadurch gekennzeichnet, daß** man ein Gemisch aus zwei verschiedenen ungesättigten Alkoholen der allgemeinen Formel II mit einem Acetessigsäurealkylester der allgemeinen Formel III zu einem Gemisch der entsprechenden ungesättigten Ketone der allgemeinen Formel I umsetzt und dieses Gemisch anschließend destillativ aufarbeitet.

12. Verfahren gemäß Anspruch 1, **dadurch gekennzeichnet, daß** man das über den Sumpf (20) der Fraktionierkolonne abgetrennte und über die Produktleitung (26) ausgeschleuste Rohprodukt zur Aufarbeitung entweder direkt oder nach Abtrennen des Gemisches (30) aus dem Katalysator und Hochsiedern mittels eines Verdampfers (31) in einer nachgeschalteten Trennwandkolonne (33) auftrennt.

## Claims

1. A process for the continuous preparation of unsaturated ketones of the formula I 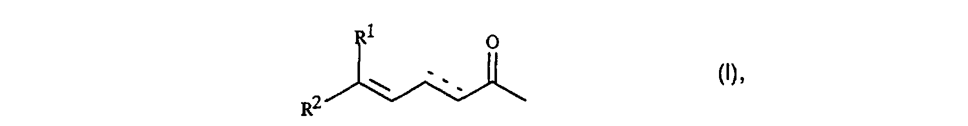 where the dashed line can be an additional C-C bond, R¹ is a branched or unbranched alkyl having from 1 to 4 carbons and R² is a saturated or unsaturated, branched or unbranched, unsubstituted or methoxy-substituted aliphatic hydrocarbon radical having 1 to 37 carbons, a cycloalkyl having from 4 to 12 carbons or a cycloalkyl alkyl having 5 to 30 carbons, by reacting an unsaturated alcohol of the formula II 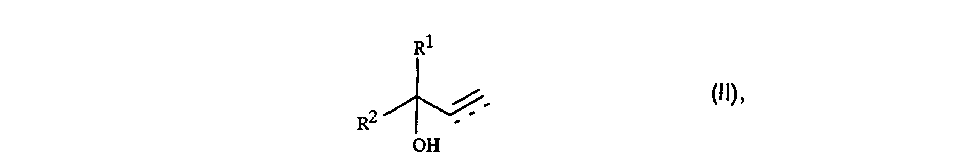 where R¹ and R² have the meanings given above,
with an alkyl acetoacetate of the formula III 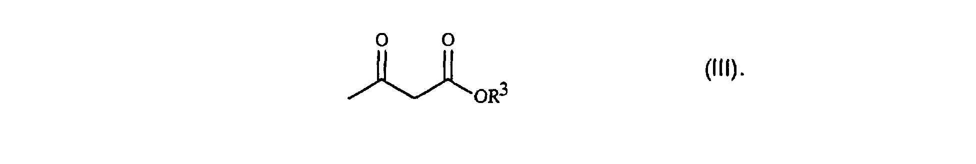 where R³ is a branched or unbranched alkyl having from 1 to 5 carbons, in the presence of organic aluminum compounds as catalyst which comprises
A. reacting the unsaturated alcohol of the formula II with the alkyl acetoacetate of the formula III on the internals (16) of a fractionation tower (4) to give the acetoacetate of the unsaturated alcohol and separating off the resultant alcohol of the formula IV
R³-OH (IV) (IV)
with the overhead stream (5) of the fractionation tower and
B. rearranging the resultant acetoacetate of the alcohol of the formula II into the unsaturated ketone of the formula I in the presence of the organic aluminum compound in the lower part of the fractionation tower and/or in the bottom (20) of the fractionation tower, the carbon dioxide forming in this case passing via the fractionation tower (4) into the overhead stream (5) and the resultant unsaturated ketone of the formula I being separated off from the fractionation tower via the bottom (20).

2. A process as claimed in claim 1, wherein the stage B. of the reaction is carried out in the presence of from 0.1 to 5 mol%, based on the alkyl acetoacetate to be reacted, of an organic aluminum compound of the formula V 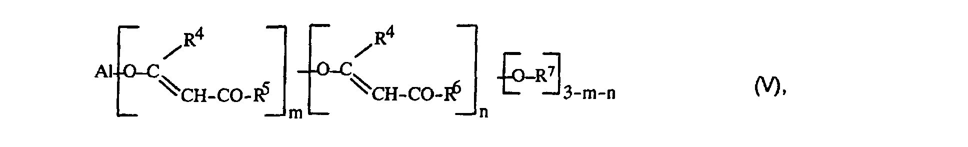 where R⁴ is branched or unbranched alkyl or alkoxy having from 1 to 4 carbons, preferably methyl or ethyl, R⁵ and R⁶ are branched or unbranched alkyl or alkoxy having from 1 to 5 carbons, preferably methyl or 2-butyl, R⁷ is a branched or unbranched alkyl having from 1 to 4 carbons and m and n can be integers from 0 to 3, where n+m ≤ 3, or is carried out in the presence of aluminum triaryloxylates as catalyst.

3. A process as claimed in claim 1, wherein, as unsaturated alcohol of the formula II, use is made of one in which R¹ is CH₃ and R² is a group of the formula VI 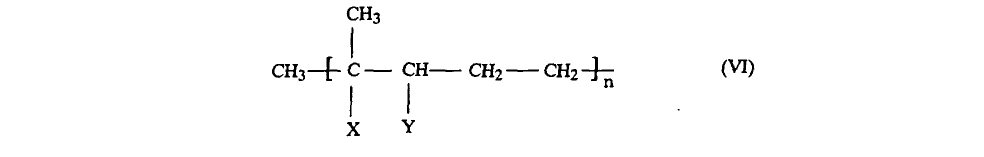 where n is an integer from 1 to 6 and X and Y are either both H, or X is methoxy and Y is H, or X and Y together are an additional bond between the X- and Y-bearing carbons.

4. A process as claimed in claim 1, wherein the starting amounts are chosen in such a manner as to give a molar ratio of alcohol of the formula II to acetoacetic ester of the formula III from 0.8 to 1.2.

5. A process as claimed in claim 1, wherein the higher-boiling reactant is fed into the fractionation tower (4) above the lower-boiling reactant.

6. A process as claimed in claim 1, wherein the catalyst is fed in liquid form into the bottom (20) of the fractionation tower (4).

7. A process as claimed in claim 1, wherein the pressure at the tower top is set in such a manner that the temperature in the bottom (20) is from 120 to 300°C.

8. A process as claimed in claim 1, wherein the residence time in the reactor system consisting of tower bottom (20) and fractionation tower (4) with or without vessel (22) is from 15 minutes to 6 hours.

9. A process as claimed in claim 1, wherein, as internals for the fractionation tower (4), use is made of tower trays, where the trays (16) are to make possible a long liquid residence time.

10. A process as claimed in claim 1, wherein heat is supplied to the reactor system consisting of tower bottom (20) and fractionation tower (4) with or without vessel (22) not only via the evaporator (12), but, in addition, via external heat exchangers (17) or via heat exchangers situated directly on the trays (16).

11. A process as claimed in claim 1, wherein a mixture of two different unsaturated alcohols of the formula II is reacted with an alkyl acetoacetate of the formula III to give a mixture of the corresponding unsaturated ketones of formula I and this mixture is then worked up by distillation.

12. A process as claimed in claim 1, wherein the crude product separated off via the bottom (20) of the fractionation tower and ejected via the product line (26) for workup is fractionated in a downstream dividing-wall tower (33) either directly or after separating off the mixture (30) from the catalyst and high-boilers by means of an evaporator (31).

## Revendications

1. Procédé pour la préparation continue de cétones insaturées de formule générale I 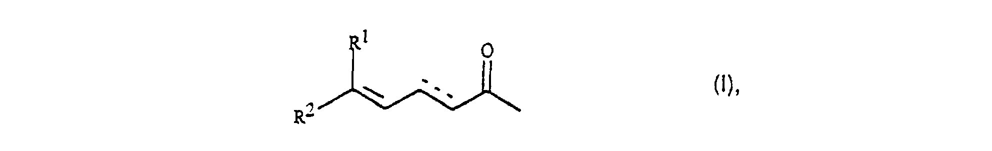 dans laquelle le trait interrompu peut représenter une liaison C-C supplémentaire, R¹ représente un groupe alkyle à chaîne droite ou ramifiée en C1-C4 et R² un radical hydrocarboné aliphatique en C1-C37, saturé ou insaturé, à chaîne droite ou ramifiée, éventuellement substitué par des groupes méthoxy, un groupe cycloalkyle en C4-C12
ou un groupe cycloalkylalkyle en C5-C30, par réaction d'un alcool insaturé de formule générale II 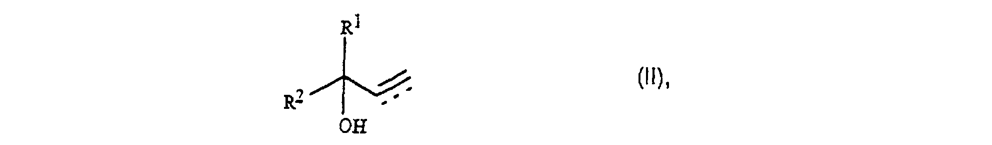 dans laquelle R¹ et R² ont les significations indiquées ci-dessus,
avec un acétylacétate d'alkyle de formule générale III 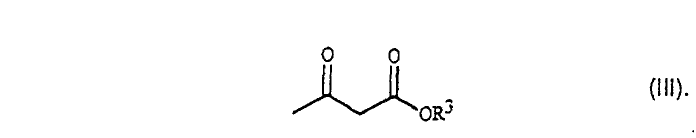 dans laquelle R³ représente un groupe alkyle à chaîne droite ou ramifiée en C1-C5, en présence d'un dérivé organique de l'aluminium qui sert de catalyseur,
**caractérisé par le fait que**
A. on fait réagir l'alcool insaturé de formule II avec l'acétylacétate d'alkyle de formule III sur les éléments internes (16) d'une colonne de fractionnement (4) avec formation de l'acétylacétate de l'alcool insaturé et séparation de l'alcool formé, répondant à la formule générale IV
R -OH (IV)
avec le courant de tête (5) de la colonne de fractionnement, et
B. on soumet l'acétylacétate de l'alcool de formule II, formé en A., à transposition dans la partie inférieure de la colonne de fractionnement et/ou dans le pied (20) de la colonne de fractionnement en la cétone insaturée de formule I, en présence du dérivé organique de l'aluminium, le dioxyde de carbone formé dans ces conditions passant, en traversant la colonne de fractionnement (4) dans le courant de tête (5), et la cétone insaturée de formule générale I, formée dans la transposition, étant séparée au pied (20) de la colonne de fractionnement.

2. Procédé selon la revendication 1, **caractérisé par le fait que** le stade B. de réaction est réalisé en présence de 0,1 à 5 mol %, par rapport à l'acétylacétate d'alkyle soumis à la réaction, d'un dérivé organique de l'aluminium de formule générale V 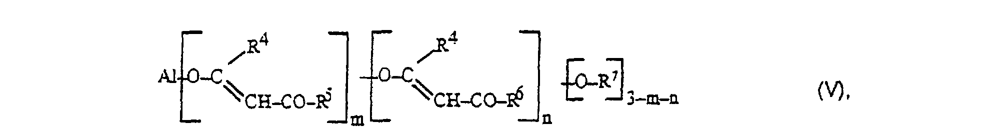 dans laquelle les symboles R⁴ représentent des groupes alkyle ou alcoxy à chaîne droite ou ramifiée en C1-C4, de préférence des groupes méthyle ou éthyle, R⁵ et R⁶ représentent des groupes alkyle ou alcoxy à chaîne droite ou ramifiée en C1-C5, de préférence des groupes méthyle ou 2-butyle, R⁷ représente un groupe alkyle à chaîne droite ou ramifiée en C1-C4 et m et n sont des nombres entiers allant de 0 à 3, avec n + m ≤ 3, ou bien en présence de triaryloxylates d'aluminium, servant de catalyseurs.

3. Procédé selon la revendication 1, **caractérisé par le fait que** l'alcool insaturé de formule générale II qui est mis en oeuvre est un alcool pour lequel R¹ représente CH₃ et R² un groupe de formule générale VI 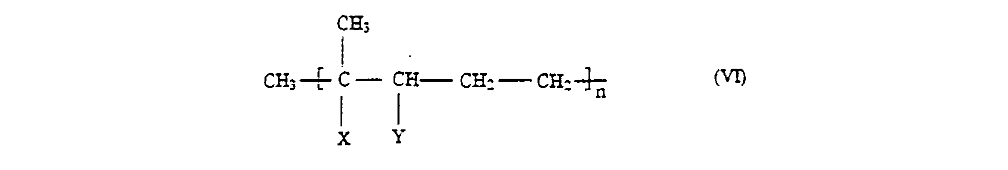 dans laquelle n est un nombre entier allant de 1 à 6 et x et y représentent tous deux H ou bien x représente un groupe méthoxy et y représente H, ou bien encore x et y représentent ensemble une liaison supplémentaire entre les atomes de carbone portant les substituants X et Y.

4. Procédé selon la revendication 1, **caractérisé par le fait que** les quantités mises en oeuvre correspondent à un rapport molaire de 0,8 à 1,2 entre l'alcool de formule II et l'ester acétylacétique de formule III.

5. Procédé selon la revendication 1, **caractérisé par le fait que** l'on introduit les réactifs à haut point d'ébullition dans la colonne de fractionnement (4) au-dessus des réactifs à bas point d'ébullition.

6. Procédé selon la revendication 1, **caractérisé par le fait que** l'on introduit le catalyseur à l'état liquide au pied (20) de la colonne de fractionnement (4).

7. Procédé selon la revendication 1, **caractérisé par le fait que** l'on règle la pression en tête de colonne en sorte que la température au pied (20) se situe entre 120 et 300°C.

8. Procédé selon la revendication 1, **caractérisé par le fait que** la durée de passage dans la partie d'appareillage consistant en le pied de colonne (20), la colonne de fractionnement (4) et le cas échéant le récipient (22) va de 15 minutes à 6 h.

9. Procédé selon la revendication 1, **caractérisé par le fait que** les éléments internes de la colonne de fractionnement (4) sont des plateaux de colonne (16) qui permettent une longue durée de passage du liquide.

10. Procédé selon la revendication 1, **caractérisé par le fait que** l'apport de chaleur à la partie d'appareillage consistant en le pied de colonne (20), la colonne de fractionnement (4) et le cas échéant le récipient (22) n'est pas dû uniquement au vaporiseur (12) mais en outre à des échangeurs de chaleur extérieurs (17) ou bien à des échangeurs de chaleur qui se trouvent directement sur les plateaux (16).

11. Procédé selon la revendication 1, **caractérisé par le fait que** l'on convertit un mélange de deux alcools insaturés différents de formule générale II, par réaction avec un acétylacétate d'alkyle de formule générale III, en un mélange des cétones insaturés correspondantes de formule générale I qu'on traite ensuite par distillation.

12. Procédé selon la revendication 1, **caractérisé par le fait que** le produit brut séparé au pied (20) de la colonne de fractionnement et évacué par le conduit de produit (26) est envoyé dans une colonne à parois de séparation (33) soit directement, soit après séparation du mélange (30) du catalyseur et des produits à haut point d'ébullition à l'aide d'un vaporiseur (31).
